# EUROPEAN PATENT SPECIFICATION

(11) **EP 3 398 648 B1**
(45) Date of publication and mention of the grant of the patent: **02.10.2019**
(21) Application number: 18167908.5
(22) Date of filing: 18.04.2018
(51) Int. Cl.: A61N 1/04, A61N 1/40

(54) **PILLOW**
KISSEN
COUSSIN

(30) Priority: 19.04.2017 IT 201700042504
(43) Date of publication of application: 07.11.2018
(73) Proprietor: Fontanesi, Franco, 42121 Reggio Emilia (IT)
(72) Inventor: BORDIGNON, Francesca, 42047 Rolo (Reggio Emilia) (IT)
(74) Representative: Casadei, Giovanni

(56) References cited:
- WO-A1-2009/043196
- DE-A1- 3 719 084
- US-A- 434 024
- US-A1- 2003 176 908

## Description

### Technical Field

The present invention pertains to the sector of electronic devices for medical applications and it specifically concerns a pillow for electromagnetic therapy.

### Prior art

Electromagnetic therapy (EMT) is a therapy that uses magnetic fields of various types to produce beneficial effects on health.

Electromagnetic therapy is applied to a subject by affixing in proximity to the subject a means for emitting electromagnetic waves typically produced by a square wave generator, the frequency of which can be selected as desired.

It is held that magnetic therapy regulates cellular electrochemical equilibrium and restores correct permeability of the cellular membrane. For this reason, electromagnetic therapy can be applied for diseases of the muscular, joint, bone and tissue systems in general.

There are known braces equipped with magnets or electromagnets that are adapted to being applied on one or more parts of the body and that enable relatively long magnetic therapy or electromagnetic therapy sessions to be carried out.

There are also known therapy tables equipped with transducer means adapted to make it possible to provide electromagnetic therapy, which, however, do not offer significant effectiveness.

Further example of this prior art is disclosed in document US 434024. WO 2009/043196 A1 discloses an electrode structure for electrotherapy.

The Applicant holds that the effectiveness of electromagnetic therapy is greater the greater the duration of the session. Specifically, an electromagnetic therapy session should last for a number of hours and substantially this is hardly compatible with conventional medical sessions in which electromagnetic therapy has a maximum duration of one hour or slightly more than one hour.

The aim of the present invention is to disclose a pillow for electromagnetic therapy that makes it possible to overcome the drawbacks described hereinabove.

### Summary of the invention

According to the present invention, a pillow is realized for electromagnetic therapy according to claim 1. Preferred embodiments are set out in claims 2-4.

According to the present invention, a device is also realized for providing electromagnetic therapy, said device comprising a generator of electrical signals and a pillow for electromagnetic therapy according to any one of claims 1-4, wherein said generator of electrical signals and said pillow are removably connectable to each other by means of an electrical conductor.

In particular, said frequency generator device is characterized in that it generates a combined high- and low-frequency electrical signal.

### Description of the figures.

A preferred and non-limiting embodiment of the invention shall now be described with reference to the attached figures, of which:
- Figure 1 is a front view of the pillow for electromagnetic therapy constituting the object of the present invention;
- Figure 2 is a sectional view of the pillow for electromagnetic therapy constituting the object of the present invention, as observed from the cut made along lines II-II of Figure 1;
- Figure 3 is a sectional view of the pillow for electromagnetic therapy constituting the object of the present invention, as observed from the cut made along lines III-III of Figure 1;
- Figure 4 is a detail of part of the pillow for electromagnetic therapy constituting the object of the present invention.

### Detailed description of the invention.

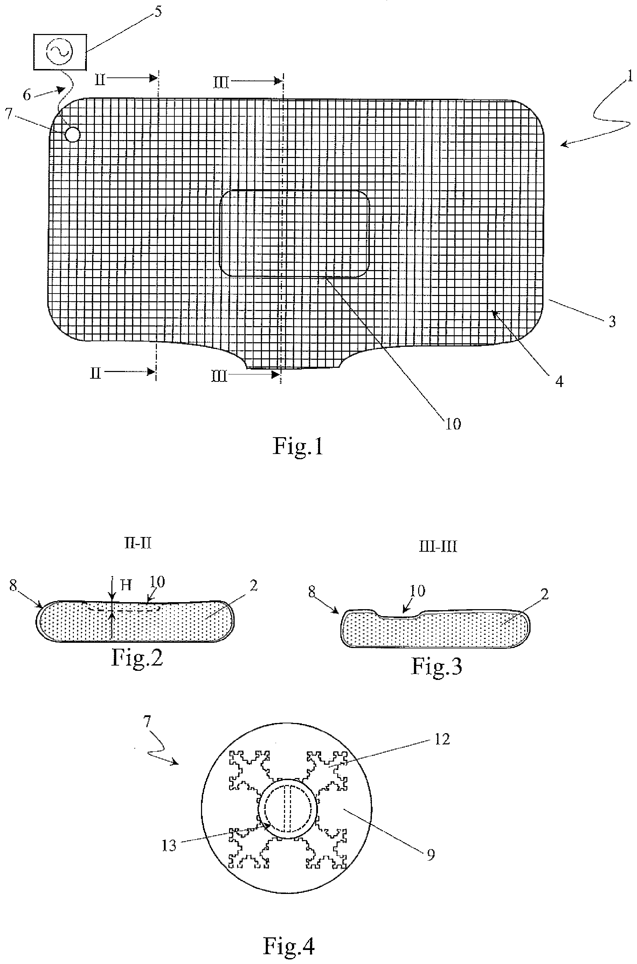

A pillow for electromagnetic therapy is indicated in its entirety in Figure 1 by reference number 1, said pillow having a padding 2 contained inside a covering sheath 3 on which, when in use, the user rests his/her head and/or neck. As illustrated in Figure 1, the covering sheath 3 and the underlying padding 2 have a substantially rectangular shape, which, however, can vary according to the wishes of the manufacturer and due to any deformation during use, which is also made possible by its flexibility. Specifically for this reason, the pillow for electromagnetic therapy described herein is illustrated in Figure 1 in a shape that is intentionally not symmetric, precisely to indicate the possibility of at least temporary deformation.

In particular, the covering sheath 3 is made of a substantially woven material comprising a system of metal conductors 4, which preferably, though not limitedly, are realized with a circular section and take on the appearance of a system of electrical wires. Even more preferably, though not limitedly, said wires are made of enamelled copper and they are interwoven in the warp and weft of said fabric. Preferably, though not limitedly, said fabric is a natural fabric or a natural and synthetic blend, possibly treated with an anti-odour material or also with anti-allergenic substances of known types.

For the purpose of ensuring correct propagation of the electromagnetic waves for magnetic therapy, the system of metal conductors 4 extends substantially over the entire surface of the sheath 3.The user can therefore assume virtually any position on said pillow, without this leading to less effectiveness of said electromagnetic therapy.

When in use, the system of metal conductors 4 is supplied with an electrical frequency signal, preferably, though not limitedly, comprised in the range between 100 Hz and 5 kHz if at low frequency or in the range between 20-30 MHz if at high frequency. In fact, this electrical signal makes the system of metal conductors 4 a sort of antenna that emits the magnetic radiation over an extensive surface and in use, the magnetic radiation flows into the neck and head of the user.

Even more preferably, said generator of electrical signals transmits a composite electrical signal which has a first component that is modulated at low frequency and a second component modulated at high frequency, which advantageously optimizes the efficiency of the electromagnetic therapy provided.

The electrical signal is generated by a known type of source or generator of electrical signals 5 and it is propagated towards the pillow 1, constituting the object of the present invention, through an electrical conductor 6 that can be disconnected from the pillow.

As illustrated in Figure 1 and in further detail in Figure 4, precisely for this reason, the pillow 1 constituting the object of the present invention advantageously comprises electrical connection means 7 for connection with the generator of electrical frequency signals 5, which, in use, supplies said electrical signal to said pillow. In particular, the electrical connection means 7 is of a removable type.

In a preferred and non-limiting embodiment of the present invention, which is illustrated in Figure 4, the electrical connection means 7 comprises an automatic button 13, which projects out on an outer face 8 of the covering sheath 3 and is connected directly to a substantially planar support 9 fixed on the inner face of the covering sheath 3. This substantially planar support 9 incorporates a system of electrical strip conductors, which is arranged according to a predetermined scheme or profile that is not movable, and the support 9 realizes a patch type of antenna 12, located substantially in contact with the system of metal conductors 4. The patch antenna is particularly indicated for emitting electrical signals in the form and frequencies indicated above. The patch antenna rests directly on the surface of the covering sheath 3 so that propagation of the electrical signal for electromagnetic therapy can take place in the best possible manner. The automatic button 13 extends on the opposite side of the support 9 and for example it is a male button adapted to coupling with a corresponding automatic female button present on the end of the opposite electrical connector 6 with respect to the one connected with the signal generator 5. As illustrated in Figures 1 to 3, for the purpose of facilitating correct positioning of the head on the pillow, the padding 2 comprises a recess 10 adapted to define a concavity for resting the back of the user's neck. This recess 10 is of a depth H in keeping with the mean nape-neck distance ratio and it is realized in the shape of a quadrangle and with rounded corners. The covering sheath 3 is glued to the padding 2 for the purpose of preventing mechanical stress on the electrical conductors, which could damage them, locally interrupting the passage of the electric current, at least at the recess. The recess 10 is found in a substantially central zone of the area of the pillow for electromagnetic therapy constituting the object of the present invention. However, this position is not to be understood as limiting.

Lastly, the padding of the pillow constituting the object of the present invention has a plurality of holes adapted to improve the passage of said electromagnetic waves.

The advantages of the pillow constituting the object of the present invention are evident in light of the preceding description. Namely, it makes it possible to take advantage of the benefits of electromagnetic therapy for a significantly longer period of time than that which is possible in a common medical session. In fact, the electromagnetic therapy provided is in this case used principally during the night sleep period, which is significantly longer - even eight hours or more - with respect to the time that is traditionally dedicated to sessions with a physician. For this reason, the effectiveness of the electromagnetic therapy is greater and it is also rendered substantially independent of the position taken by the user while sleeping.

## Claims

1. A pillow (1) for electromagnetic therapy, said pillow comprising:
- a padding (2);
- a sheath (3) for covering said padding (2) on which the user rests his/her head and/or neck when the pillow is in use;
- wherein said covering sheath (3) is made of a substantially woven material comprising a system of metal conductors (4); and
- wherein said system of metal conductors (4) extends substantially over the entire surface of said covering sheath and is suitable for being supplied with an electrical signal such as to have electromagnetic waves for electromagnetic therapy be transmitted to said system of metal conductors, and such that, in use, the waves flow into said head and/or said neck of said subject;
- electrical connection means (7) for connection with a generator of electrical frequency signals (5), which, in use, supplies said electrical signal to said pillow (1), wherein said electrical connection means (7) realizes a removable type of connection ;
wherein said electrical connection means (7) comprises an automatic button, which projects out on an outer face (8) of said covering sheath (3) and is connected directly to a substantially planar support (9) fixed on the inner face of said covering sheath (3) ;
**characterized in that** said substantially planar support (9) incorporates a patch type of antenna (12) that is in contact with said system of metal conductors.

2. The pillow (1) for electromagnetic therapy according to claim 1, wherein said padding (2) comprises a recess (10) adapted to define a concavity for resting the back of the neck of the subject.

3. The pillow (1) for electromagnetic therapy according to claim 2, wherein said sheath is glued to said padding at least at said concavity.

4. The pillow (1) for electromagnetic therapy according to claim 2, wherein said recess (10) is quadrangular in shape and it is positioned in a substantially central position of said pillow.

5. A device for providing electromagnetic therapy, comprising a generator of electrical signals (5) and a pillow (1) for electromagnetic therapy according to any one of preceding claims 1-4, wherein said generator of electrical signals (5) and said pillow (1) are removably connectable to each other by means of an electrical conductor (6).

6. The device for providing electromagnetic therapy according to claim 5, **characterized in that** it generates a high-frequency electrical signal.

## Patentansprüche

1. Kissen (1) für die elektromagnetische Therapie, wobei das Kissen umfasst:
- eine Polsterung (2);
- eine Hülle (3) zum Abdecken der Polsterung (2), auf der der/die Benutzer/in seinen/ihren Kopf und/oder Nacken aufliegt, wenn das Kissen verwendet wird;
- wobei die Abdeckhülle (3) aus einem im Wesentlichen gewebten Material hergestellt ist, das ein System aus Metallleitern (4) umfasst; und
- wobei sich das System aus Metallleitern (4) im Wesentlichen über die gesamte Oberfläche der Abdeckhülle erstreckt und dazu geeignet ist, um mit einem elektrischen Signal versorgt zu werden, um elektromagnetische Wellen für die elektromagnetische Therapie auf das System aus Metallleitern übertragen zu lassen, und so dass die Wellen im Gebrauch in den Kopf und/oder den Nacken des Subjekts fließen;
- elektrische Verbindungsmittel (7) zur Verbindung mit einem Generator für elektrische Frequenzsignale (5), der im Gebrauch das elektrische Signal an das Kissen (1) liefert, wobei die elektrischen Verbindungsmittel (7) eine entfernbare Art von Verbindung herstellen;
wobei die elektrischen Verbindungsmittel (7) einen automatischen Knopf umfassen, der an einer Außenseite (8) der Abdeckhülle (3) vorsteht und direkt mit einem im Wesentlichen ebenen Träger (9) verbunden ist, der an der Innenseite der Abdeckhülle (3) befestigt ist;
**dadurch gekennzeichnet, dass** der im Wesentlichen ebene Träger (9) eine Patch-Art von Antenne (12) enthält, die mit dem System aus Metallleitern in Kontakt steht.

2. Kissen (1) für die elektromagnetische Therapie nach Anspruch 1, wobei die Polsterung (2) eine Aussparung (10) umfasst, die angepasst ist, um eine Konkavität zum Aufliegen des Nackenrückens des Subjekts zu definieren.

3. Kissen (1) für die elektromagnetische Therapie nach Anspruch 2, wobei die Hülle mindestens in der Konkavität an die Polsterung geklebt ist.

4. Kissen (1) für die elektromagnetische Therapie nach Anspruch 2, wobei die Aussparung (10) eine viereckige Form aufweist und in einer im Wesentlichen zentralen Position des Kissens positioniert ist.

5. Vorrichtung zur Bereitstellung einer elektromagnetischen Therapie, umfassend einen Generator für elektrische Signale (5) und ein Kissen (1) für die elektromagnetische Therapie nach einem der vorhergehenden Ansprüche 1-4, wobei der Generator für elektrische Signale (5) und das Kissen (1) mittels eines elektrischen Leiters (6) lösbar miteinander verbindbar sind.

6. Vorrichtung zur Bereitstellung einer elektromagnetischen Therapie nach Anspruch 5, **dadurch gekennzeichnet, dass** sie ein hochfrequentes elektrisches Signal erzeugt.

## Revendications

1. Coussin (1) destiné à la thérapie électromagnétique, ledit coussin comprenant :
- un rembourrage (2) ;
- une gaine (3) servant à recouvrir ledit rembourrage (2) sur laquelle l'utilisateur pose sa tête et/ou son cou lorsque le coussin est utilisé ;
- dans lequel ladite gaine de recouvrement (3) est constituée d'une matière substantiellement tissée comprenant un système de conducteurs métalliques (4) ; et
- dans lequel ledit système de conducteurs métalliques (4) se prolonge substantiellement sur toute la surface de ladite gaine de recouvrement et est adapté pour être alimenté avec un signal électrique de manière à transmettre des ondes électromagnétiques pour la thérapie électromagnétique au dit système de conducteurs métalliques, et de manière à ce que, en fonctionnement, les ondes circulent dans ladite tête et/ou ledit cou dudit sujet ;
- un moyen de connexion électrique (7) pour la connexion à un générateur de signaux électriques de fréquence (5), qui, en fonctionnement, alimente ledit signal électrique au dit coussin (1), dans lequel ledit moyen de connexion électrique (7) effectue une connexion amovible ;
dans lequel ledit moyen de connexion électrique (7) comprend un bouton automatique qui dépasse hors d'un côté extérieur (8) de ladite gaine de recouvrement (3) et est relié directement à un support (9) substantiellement planaire fixé sur le côté intérieur de ladite gaine de recouvrement (3) ;
**caractérisé en ce que** ledit support (9) substantiellement planaire intègre une antenne patch (12) étant en contact avec ledit système de conducteurs métalliques.

2. Coussin (1) destiné à la thérapie électromagnétique selon la revendication 1, dans lequel ledit rembourrage (2) comprend un renfoncement (10) adapté pour définir une concavité pour mettre en appui la nuque du sujet.

3. Coussin (1) destiné à la thérapie électromagnétique selon la revendication 2, dans lequel ladite gaine est collée au dit rembourrage au moins en correspondance de ladite concavité.

4. Coussin (1) destiné à la thérapie électromagnétique selon la revendication 2, dans lequel ledit renfoncement (10) a une forme quadrangulaire et est positionné dans une position substantiellement centrale dudit coussin.

5. Dispositif prévu pour une thérapie électromagnétique, comprenant un générateur de signaux électriques (5) et un coussin (1) destinée à la thérapie électromagnétique selon l'une quelconque des revendications de 1 à 4, dans lequel ledit générateur de signaux électriques (5) et ledit coussin (1) peuvent se raccorder l'un à l'autre de façon amovible au moyen d'un conducteur électrique (6).

6. Dispositif prévu pour une thérapie électromagnétique selon la revendication 5, **caractérisé en ce qu'**il génère un signal électrique haute fréquence.
